# EUROPEAN PATENT APPLICATION

(11) **EP 4 624 043 A1**
(43) Date of publication of application: **01.10.2025**
(21) Application number: 23913003.2
(22) Date of filing: 28.12.2023
(51) Int. Cl.: B01J 23/62, B01J 35/30, B01J 35/61, B01J 35/63, B01J 35/64, B01J 37/16

(54) **HYDROGENATION CATALYST**

(30) Priority: 28.12.2022 KR 20220187904
(71) Applicant: Hanwha Solutions Corporation, Jung-gu Seoul 04541 (KR)
(72) Inventor: KIM, Jeong Kwon, Daejeon 34128 (KR); PARK, Woo Jin, Daejeon 34128 (KR); LEE, Byungjin, Daejeon 34128 (KR); JEON, Bongsik, Daejeon 34128 (KR)
(74) Representative: Berggren Oy
(86) International application number: PCT/KR2023/021943
(87) International publication number: WO 2024/144329

(57) **Abstract**

Provided are a catalyst for hydrogenation reaction, and more specifically, to a hydrogenation catalyst that enables high selectivity and conversion rate in a hydrogenation reaction that converts a carboxylic acid group, an aldehyde group, or a ketone group into an alcohol group.

## Description

### [TECHNICAL FIELD]

### CROSS-REFERENCE TO RELATED APPLICATION(S)

This application claims the benefit of priority based on Korean Patent Application No. 10-2022-0187904, filed on December 28, 2022, and all contents disclosed in the documents of the aforementioned Korean patent applications are comprised as a part of this specification.

The present invention relates to a hydrogenation catalyst, and more specifically, to a hydrogenation catalyst having high selectivity and conversion rate in a hydrogenation reaction to convert a carboxylic acid group, an aldehyde group, or a ketone group into an alcohol group.

### [BACKGROUND]

Generally, a compound comprising an alcohol group corresponds to an organic compound having a hydroxyl group, and a great number of alcohol compounds are very important compounds widely used in various industrial fields.

Among these, examples of alcohols having one hydroxyl group (monohydric alcohols) are methanol, ethanol, n-propanol, and the like. Many monohydric alcohols are frequently used as raw materials for chemical synthesis or as solvents. Furthermore, examples of alcohols having two hydroxyl groups (dihydric alcohols or diols) are ethylene glycol, propylene glycol, 1,4-butanediol, and the like. Such diol compounds are widely used as various basic industrial materials for polyesters, polyurethanes, varnishes, adhesives, pharmaceuticals, and the like.

Many of these important alcohol compounds are industrially mass-produced. In particular, as a method for industrially producing primary alcohols (alcohols having a hydroxymethyl group, -CH2OH), for example, a method of hydrating olefins is known, and is mainly used for ethanol production. In addition, for the production of alcohols having 3 or more carbon atoms other than ethanol, such as n-propanol, n-butanol or 1,4-butanediol, a method of hydrogenating carboxylic acid esters in the presence of a copper-containing catalyst under high temperature and high pressure conditions is known. However, in the existing hydrogenation reaction, after producing an esterified compound from a carboxylic acid, the prepared ester compound undergoes a hydrogenation reaction, which inevitably complicates the primary alcohol manufacturing process.

Furthermore, recently, the demand for environmentally friendly and biodegradable diol compounds is significantly increasing. These diol compounds may be converted from dicarboxylic acids or their derivatives through a hydroprocessing catalyst. Most of the existing catalyst processes that enable this are catalyst processes based on Cu-Cr or Zr-Cr, or catalyst processes based on ruthenium oxide, ruthenium-carbon composites, etc., but the catalyst processes described above require high pressure (200-300 bar) operating conditions.

In addition, Mitsubishi Chemical Corporation (MCC) and Asahi Kasei have patented a RuPtSn/C catalyst as a dicarboxylic acid-to-diol catalyst (US6294703, US6495730), but Pt has the disadvantage of being a metal with higher price volatility and higher cost than Ru. Lotte Chemical also filed a patent (WO 2015-156582) by supporting the same metal (RuPtSn) on Y-zeolite, but it also incurs high catalyst costs, and there is the inconvenience of additionally introducing a delaminated process when applied to hydrothermal reactions.

Accordingly, there is a need for the development of a catalyst that can efficiently perform a hydrogenation reaction of carboxylic acids or carboxylic acid esters under relatively low pressure conditions without excessive reaction time or high temperature conditions, even without using expensive noble metals such as platinum (Pt).

### [PRIOR ART DOCUMENTS]

### [Patent Documents]

Japanese Patent Publication No. 1995-165644
Chinese Patent Publication No. 001911504
U.S. Patent Publication No. 6294703
U.S. Patent Publication No. 6495730
International Patent Publication No. WO 2015-156582

### [DETAILED DESCRIPTION OF THE INVENTION]

### [Technical Problem]

There is provided a hydrogenation catalyst that enables high selectivity and conversion rate in a hydrogenation reaction that converts a carboxylic acid group, an aldehyde group, or a ketone group into an alcohol group, under relatively low pressure conditions without excessive reaction time or high temperature conditions.

There is also provided a process for preparing the hydrogenation catalyst.

### [Technical Solution]

According to an embodiment of the present invention, a hydrogenation catalyst is provided, comprising ruthenium and tin as catalytically active metal(s),
wherein the content of ruthenium is 30 wt% or more and less than 55 wt%, and the content of tin is greater than 45 wt% and 70 wt% or less, based on the total weight of ruthenium and tin, and
wherein the hydrogenation catalyst has a hydrogen adsorption ratio (A2/A1) of 70 mol% to 99 mol%, and the hydrogen adsorption ratio (A2/A1) is the amount of hydrogen adsorption (A2) at a temperature of 523 K or less of the hydrogenation catalyst to the total amount of hydrogen adsorption (A1) of the hydrogenation catalyst, as measured by a Hydrogen-Temperature-Programed Reduction (H₂-TPR) analysis.

According to another embodiment of the present invention, a process for preparing the hydrogenation catalyst is provided.

According to further another embodiment of the present invention, a hydrogenation method performing a hydrogenation reaction using the above-described catalyst is provided.

### [ADVANTAGEOUS EFFECTS]

According to the present invention, the hydrogenation catalyst shows an excellent effect of enabling high selectivity and conversion rate, simultaneously, in a hydrogenation reaction to convert a carboxylic acid group, an aldehyde group, or a ketone group into an alcohol group, under relatively low pressure conditions without excessive reaction time or high temperature conditions.

### [BRIEF DESCRIPTION OF THE DRAWINGS]

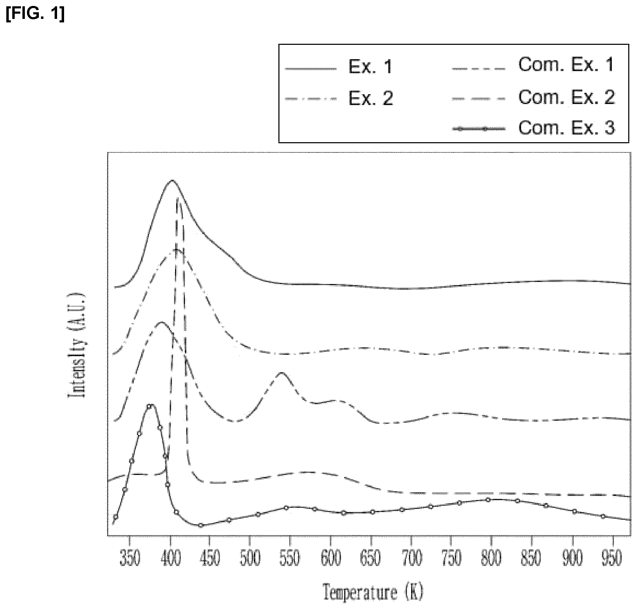

FIG. 1 shows the hydrogen adsorption curves of the passivation layer according to Hydrogen-Temperature-Programed Reduction (H₂-TPR) analysis for Examples 1 to 2 (Exs. 1 to 2) and Comparative Examples 1 to 3 (Com. Exs. 1 to 3) according to an embodiment of the present disclosures.

### [DETAILED DESCRIPTION OF THE EMBODIMENTS]

The terms used in this description are just for explaining exemplary embodiments and it is not intended to restrict the present invention. The singular expression may include the plural expression unless it is differently expressed contextually.

In this specification, terms such as "comprising", "having", or "including " are for describing the implemented features, numbers, steps, components, or combinations thereof, and do not exclude the possibility of one or more other features, numbers, steps, components, combinations thereof, or additions.

In addition, terms of degree such as "about", "substantially", etc., used throughout this specification are used in a meaning that is at or close to the numerical value when inherent manufacturing and material tolerances are presented for the stated meaning, and are used to prevent unscrupulous infringers from unduly exploiting disclosed contents where accurate or absolute numerical values are mentioned to aid in understanding of the present application.

For reference, "parts by weight", as used herein, refers to a relative concept of a ratio of the weight of the remaining material based on the weight of a specific material. For example, in a mixture containing 50 g of material A, 20 g of material B, and 30 g of material C, the amounts of material B and material C based on 100 parts by weight of material A are 40 parts by weight and 60 parts by weight, respectively.

Meanwhile, "wt% (% by weight)" refers to an absolute concept of expressing the weight of a specific material in percentage based on the total weight. In the above-exemplified mixture, the contents of material A, material B, and material C based on 100% of the total weight of the mixture are 50% by weight, 20% by weight, and 30% by weight, respectively. At this time, a sum of contents of the respective components does not exceed 100% by weight.

The present invention may be variously modified and have various forms, and specific embodiments will be illustrated and described in detail as follows. It should be understood, however, that the description is not intended to limit the present invention to the particular forms disclosed, but on the contrary, the intention is to cover all modifications, equivalents, and alternatives falling within the spirit and scope of the invention.

Hereinafter, the hydrogenation catalyst of the present invention, a process for preparing the same, and a hydrogenation method using the same will be described in more detail.

### Hydrogenation Catalyst

According to an embodiment of the present disclosures, a hydrogenation catalyst is provided that enables high selectivity and conversion rate in a hydrogenation reaction that converts a carboxylic acid group, an aldehyde group, or a ketone group into an alcohol group, under relatively low pressure conditions without excessive reaction time or high temperature conditions.

The hydrogenation catalyst according to an embodiment of the present disclosures comprises ruthenium and tin as catalytically active metal(s).

Specifically, the hydrogenation catalyst is characterized in that the content of ruthenium is 30 wt% or more and less than 55 wt% based on the total weight of ruthenium and tin, the content of tin is greater than 45 wt% and 70 wt% or less based on the total weight of ruthenium and tin. Also, the hydrogenation catalyst has a hydrogen adsorption ratio (A2/A1) of 70 mol% to 99 mol%, and the hydrogen adsorption ratio (A2/A1) is the amount of hydrogen adsorption (A2) at a temperature of 523 K (kelvin temperature) or less of the hydrogenation catalyst to the total amount of hydrogen adsorption (A1) of the hydrogenation catalyst, as measured by a Hydrogen-Temperature-Programed Reduction (H₂-TPR) analysis.

In particular, the hydrogenation catalyst according to an embodiment of the present disclosures is a catalyst aiming for ruthenium and tin, which are catalytically active metal(s), to form a homogeneous alloy status while being comprised within a specific range. Generally, as a hydrogenation catalyst, platinum (Pt) among noble metals is known as a metal that has high hydrogen adsorption capacity, promotes the reduction of Sn species, and improves catalyst performance and stability by preventing the leaching out of Sn that occurs during hydrothermal reactions. However, the hydrogenation catalyst described above shows even better hydrogenation reaction results without using such expensive platinum (Pt) and has the characteristic that no leaching of Sn occurs during the reaction.

Specifically, in the hydrogenation catalyst, the reduction mechanism of the carboxylic acid group on the composite metal (Ru-Sn) phase is as follows. Ruthenium (Ru) metal adsorbs hydrogen to form a metal-hydrogen compound (metal-hydride), and tin (Sn) acts as a Lewis acid site to activate the carboxylic acid group. Subsequently, the metal-hydrogen compound (metal-hydride) binds to the activated carboxylic acid group, aldehyde group, or ketone group and is converted into an alcohol group.

Meanwhile, in the case of a selective hydrogenation reaction that converts a carboxylic acid group (-(C=O)OH), an aldehyde group (-(C=O)H), or a ketone group (-(C=O)-) to an alcohol group (-OH) as in the present disclosures, the characteristics of the metal change as the metal component changes, resulting in differences in hydrogen adsorption strength, adsorption amount, and the like. In fact, when comparing Ru-Sn and Ni-Sn metals used for similar carboxylic acid group reduction reactions, using Ni metal may lead to problems requiring more reaction time and higher reaction temperature under similar conditions. This is a result of the superior hydrogen adsorption capacity of Ru metal compared to Ni metal.

Specifically, in the hydrogenation catalyst, the content of ruthenium is 30 wt% or more and less than 55 wt% based on the total weight of ruthenium and tin, and the content of tin is greater than 45 wt% and 70 wt% or less based on the total weight of ruthenium and tin. More specifically, the ruthenium content may be 30.5 wt% or more, or 30.8 wt% or more, and 54.5 wt% or less, or 53 wt% or less, or 50 wt% or less, or 47 wt% or less. The tin content may be 45.5 wt% or more, or 47 wt% or more, or 50 wt% or more, or 53 wt% or more, and 69.5 wt% or less, or 69.2 wt% or less.

In addition, the hydrogenation catalyst according to an embodiment of the present disclosures is characterized in that ruthenium as the catalytically active metal(s) is comprised in a range effective for catalytic action. Specifically, ruthenium as the catalytically active metal(s) may be comprised in an amount of 1 part by weight or more and 11 parts by weight or less based on the total weight of the catalyst. More specifically, ruthenium as the catalytically active metal(s) may be comprised in an amount of 1.5 parts by weight or more and 10.5 parts by weight or less, or 2 parts by weight or more and 10 parts by weight or less, or 3.5 parts by weight or more and 9.5 parts by weight or less, or 4.5 parts by weight or more and 9 parts by weight or less, or 4.8 parts by weight or more and 8 parts by weight or less, or 5.0 parts by weight or more and 7.5 parts by weight or less, or 5.1 parts by weight or more and 7 parts by weight or less based on the total weight of the catalyst. **In** particular, when ruthenium is comprised in a high content, namely, when ruthenium is comprised in an amount exceeding 11 parts by weight, there is a disadvantage of an increased catalyst price. In addition, there may be a problem of additional process costs due to a decrease in the selectivity of the target product as the size of the active metal comprising ruthenium and tin, which constitute the catalyst, increases. Furthermore, when ruthenium is comprised in an amount less than 1 part by weight, there may be a problem of decreased conversion efficiency and thus reduced production efficiency of an alcohol compound such as 1,4-butanediol (1,4-BDO) in the hydrogenation reaction.

Meanwhile, tin as the catalytically active metal(s) may be comprised in an amount of 1 part by weight to 12 parts by weight, or 1.5 parts by weight to 11 parts by weight, or 2 parts by weight to 10.8 parts by weight, or 4.5 parts by weight to 10.5 parts by weight, or 5 parts by weight to 10.2 parts by weight, or 5.5 parts by weight to 10 parts by weight, or 5.9 parts by weight to 9.8 parts by weight based on the total weight of the catalyst.

Also, the tin among the catalytically active metal(s) may be comprised in a weight ratio of 0.1 to 3.0, or 0.5 to 2.8, or 0.8 to 2.67, or 0.9 to 2.5, or 1.0 to 2.4, or 1.1 to 2.3 relative to the ruthenium on a weight basis.

**It** is preferable that the hydrogenation catalyst according to an embodiment of the present disclosures comprises ruthenium and tin as catalytically active metal(s) in the ranges described above. If the content is comprised below the ranges described above, the conversion efficiency of the reaction may decrease or the selectivity of the target product may decrease, leading to excessive separation and recovery costs in the process. That is, if the content of ruthenium among the catalytically active metal(s) in the hydrogenation catalyst is less than 1 part by weight, when performing a hydrogenation reaction of a carboxylic acid group, an aldehyde group, or a ketone group to an alcohol group, the conversion efficiency to a compound comprising an alcohol group, for example, 1,4-butanediol (BDO), decreases. On the other hand, if the content exceeds the ranges described above, the dispersibility of the metal decreases, and the crystal size increases, which may also result in a decrease in conversion efficiency.

Meanwhile, the particle size of the active metal in the hydrogenation catalyst may be about 3 nm to about 15 nm, and specifically, about 3.5 nm to about 14 nm, or about 4 nm to about 13 nm, or about 4.2 nm to about 12 nm, or about 4.3 nm to about 11 nm, or about 4.4 nm to about 10 nm. The particle size of the metal may be an average value of the measured particles, for example, the particle size values of 100 samples measured by a transmission electron microscope. Specifically, the average particle size of the active metal may be about 3.8 nm to about 12 nm, or about 4.0 nm to about 10 nm, or about 4.2 nm to about 9 nm, or about 4.3 nm to about 8 nm, or about 4.5 nm to about 7.5 nm. For example, the particle size of the active metal in the hydrogenation catalyst may be about 3 nm to about 6 nm, and the average particle size of the active metal may be about 4 nm to about 5 nm, or the particle size of the active metal in the hydrogenation catalyst may be about 4 nm to about 12 nm, and the average particle size of the active metal may be about 7 nm to about 8 nm.

In particular, the hydrogenation reaction using the catalyst of the present disclosures is, for example, a conversion reaction to a linear alcohol through the selective hydrogenation reaction of a di-carboxylic acid. In such a hydrogenation reaction, the role of the catalyst is to act as a mediator that adsorbs hydrogen, which is a reducing agent, and supplies it to the reactants. If smooth hydrogen transfer does not occur, a reverse reaction (oxidation reaction) of the converted product may occur. Therefore, in order for the forward hydrogenation reaction to occur efficiently, it is preferable that ruthenium and tin, which are catalytically active metal(s), are comprised within the content ranges as described above.

Meanwhile, the hydrogenation catalyst according to an embodiment of the present disclosures is characterized in that, as described above, ruthenium and tin, which are catalytically active metal(s), are comprised within a specific range, and ruthenium and tin form a homogeneous alloy status, while the ratio (A2/A1) of the amount of hydrogen adsorption (A2) at a temperature of 523 K or less to the total amount of hydrogen adsorption (A1) of the catalyst, as measured by H₂-TPR (Hydrogen-Temperature Program Reduction) analysis, is 70 mol% to 99 mol%.

Here, the H₂-TPR (temperature programmed reduction) analysis is an analysis that measures the behavior of gases desorbing/adsorbing depending on the temperature of the catalyst, and the adsorption behavior characteristics may vary depending on the type of metal, the amount of supporting, and the distribution form of the metal. In particular, even if ruthenium and tin satisfy a specific weight ratio in the catalyst, if the metal is not in an alloy status but is segregated, the catalyst activity may sharply decrease.

Specifically, for the hydrogenation catalyst, the ratio (A2/A1) of the amount of hydrogen adsorption (A2) at a temperature of 523 K or less to the total amount of hydrogen adsorption (A1) of the catalyst, as measured by H₂-TPR (Hydrogen-Temperature Program Reduction) analysis, may be 75 mol% or more, or 80 mol% or more, or 82 mol% or more, or 85 mol% or more, or 87 mol% or more, and 98 mol% or less, or 97 mol% or less, or 96 mol% or less, or 95 mol% or less, or 94.5 mol% or less.

The specific method related to the H₂-TPR (Hydrogen-Temperature Program Reduction) analysis for the hydrogenation catalyst according to an embodiment of the present disclosures is as shown in Example 1, which will be described later.

For example, Hydrogen-Temperature-Programed Reduction (H₂-TPR, Hydrogen-Temperature Program Reduction) analysis is an analytical method for evaluating the amount of hydrogen adsorption adsorbed by the metal at a specific reaction temperature, and the detailed measurement method is as follows.

### <H₂-TPR Analysis Method>

### - Pre-treatment: Catalyst pre-treatment and TCD signal stabilization

① Flow inert gas (Ar or N₂) through a layer comprising the catalyst and heat to 373 K at a rate of 5 K/min;
② Maintain inert gas flow through the catalyst layer at 373 K for 30 minutes;
③ Cool to 323 K while flowing inert gas through the catalyst layer;
④ Stabilize the TCD signal by flowing a mixture of inert gas and hydrogen through the catalyst layer for 60 minutes. At this time, the volume ratio of inert gas to H2 is fixed at 1:0.05.

### - H₂-TPR Analysis

① Analytical gas: H₂/Ar 5% mixed gas at 30 sccm (standard cubic centimeter per minute)
② Heat to 1073 K at a rate of 5 K/min.

More specifically, in the present disclosures, the area corresponding to the total amount of hydrogen adsorption in moles adsorbed by the passivation layer comprising the catalyst, which is surrounded by the H₂-TPR curve and baseline from the initial temperature of 333 K to the metal reduction temperature of 973 K, may be defined as A1. The area corresponding to the amount of hydrogen adsorption in moles within the hydrogenation reaction temperature range adsorbed by the passivation layer comprising the catalyst, which is surrounded by the H₂-TPR curve and baseline from the initial temperature of 333 K to the hydrogenation reaction temperature of 523 K may be defined as A2. Then, the ratio (mol%) of A2 to A1 may be measured.

By performing H₂-TPR analysis by the method as described above, the ratio of the amount of hydrogen adsorption at a temperature of 523 K or less to the total amount of hydrogen adsorption of the catalyst may be measured.

In particular, during the hydrogenation reaction that converts a carboxylic acid group, an aldehyde group, or a ketone group into an alcohol group, the catalyst adsorbs hydrogen. At this time, hydrogen adsorbed on the catalyst above the reaction temperature does not participate in the reaction, so the amount of hydrogen adsorbing/desorbing at 523 K or less significantly affects the reaction activity. Therefore, for the hydrogenation catalyst, the amount of hydrogen adsorption at 523 K or less in the H₂-TPR graph must satisfy the range described above.

For example, from the hydrogen adsorption curve of the passivation layer for the hydrogenation catalyst, the catalyst performance when performing a hydrogenation reaction may be predicted.

Specifically, the performance of the catalyst in the present disclosures is measured in a Batch reactor for liquid phase hydrothermal reactions, so the reduction of the catalyst before activity evaluation may proceed ex-situ. In particular, since the metal-state catalyst is flammable, it may be handled after reduction and then stabilization through passivating. In particular, the catalyst according to an embodiment of the present disclosures converts reactants into target products through a hydrogenation reaction. At this time, the most important role is the hydrogen adsorption behavior characteristics.

For example, because the passivated catalyst is introduced into the reactor before the catalytic reaction, and hydrogen is pressurized after being heated to the reaction temperature (503 K, kelvin temperature), hydrogen adsorbed at temperatures above approximately 523 K cannot participate in the reaction. Additionally, in the catalytic reaction mechanism, hydrogenation occurs on the surface of the Ru-Sn metal. In other words, if the Ru-Sn does not form an alloy and instead forms separate metal phases, side products such as gamma-butyrolactone or those from gasification reactions are produced rather than conversion to the desired product, 1,4-butanediol.

Therefore, when Ru-Sn metal is well mixed, a single hydrogen adsorption peak occurs in H₂-TPR analysis, and the broadness is determined by the difference in heating rate (5 °C/min) and the size of the Ru-Sn metal.

Thereby, the hydrogen adsorption curve of the passivation layer for the hydrogenation reaction catalyst according to an embodiment of the present disclosures enables the prediction of the hydrogen adsorption curve of the composite metal (Ru-Sn alloy) of ruthenium and tin that participate in the actual catalytic reaction.

In particular, even if a similar hydrogen adsorption curve is shown through H₂-TPR (Hydrogen-Temperature Program Reduction) analysis, there may be limitations in the metal systems that can participate in the hydrogenation reaction as a hydrogenation catalyst. Generally, noble metal systems such as Pd, Ru, Ni, Pt in single catalysts show similar ranges of hydrogen adsorption behavior, but their ability to activate carbonyl groups (C=O) in reactants is not good, leading to decreased catalytic activity. That is, for an efficient reduction reaction through the hydrogenation reaction pathway of the carbonyl group, as described above, an activation reaction by an oxygen-affine metal such as tin is required to facilitate hydrogen adsorption to the carbonyl group (C=O) by a composite metal catalyst of ruthenium and tin (Ru-Sn) comprising ruthenium and tin as catalytically active metal(s) in an optimal range.

However, the hydrogenation catalyst according to an embodiment of the present disclosures may further comprise one or more metals selected from the group consisting of palladium (Pd), rhodium (Rh), platinum (Pt), iron (Fe), rhenium (Re), and gallium (Ga), in addition to the ruthenium and tin described above as catalytically active metal(s), in terms of reinforcing overall process stability or reaction efficiency. When such additional catalytically active metal(s) are comprised, for example, when Pt and the like are additionally comprised, higher performance can be obtained by promoting the reduction of Sn species and preventing the leaching out of Sn that occurs during the reaction.

When one or more additional transition metals selected from the group consisting of palladium (Pd), rhodium (Rh), platinum (Pt), iron (Fe), rhenium (Re), and gallium (Ga) are additionally contained as catalytically active metal(s) in addition to ruthenium and tin, the amount of the one or more metals may preferably be 5 or less, or 0.1 to 5, more preferably 2 or less, or 0.2 to 2, in terms of their atomic ratio to ruthenium. For example, the atomic ratio of one or more additional transition metals (ruthenium: additional transition metal atomic ratio) from palladium (Pd), rhodium (Rh), platinum (Pt), iron (Fe), rhenium (Re), and gallium (Ga) based on the ruthenium (Ru) atomic ratio may be 1:5 or less, or 1:0.001 to 1:5, preferably 1:0.1 to 1:5, and more preferably 1:2 or less, or 1:0.2 to 1:2.

However, when such additional metals are comprised, there is a disadvantage that the overall catalyst manufacturing cost increases. In particular, the hydrogenation catalyst according to an embodiment of the present disclosures is characterized in that it comprises ruthenium and tin, which are catalytically active metal(s), within a specific range, and optimizes the amount of hydrogen adsorption ratio according to H2-TPR (Hydrogen-Temperature Program Reduction) analysis to form a homogeneous alloy status, thereby achieving even better hydrogenation reaction performance without using the expensive Pt (about twice the price of Ru) as described above, and no leaching of Sn occurs during the reaction.

Accordingly, the hydrogenation catalyst according to an embodiment of the present disclosures preferably comprises only ruthenium and tin as catalytically active metal(s).

Meanwhile, the catalytically active metal(s) are supported on a porous carbon-based carrier.

According to an embodiment of the present disclosures, a composite metal catalyst of ruthenium and tin (Ru-Sn) comprising ruthenium and tin as catalytically active metal(s) within an optimal range is suitable for a porous carbon-based carrier, especially a carbon-based carrier, because the hydrogenation reaction proceeds under high-temperature hydrothermal reaction conditions during actual application. When conventionally known alumina or silica-based carriers are used, they are unstable under hydrothermal reaction conditions, causing phase transformation, which can significantly reduce catalytic activity when used as a catalyst carrier.

The porous carbon-based carrier is not particularly limited, but at least one selected from the group consisting of activated carbon, carbon black, graphite, graphene, ordered mesoporous carbon (OMC), and carbon nanotubes may be used. Preferably, it may be carbon black with a high ratio of mesopores among the total pores, and in specific examples, the activated carbon may be SX ULTRA, CGSP, PK1-3, SX 1G, DRACO S51HF, CA-1, A-51, GAS 1240 PLUS, KBG, CASP, and SX PLUS, etc., and the carbon black may be BLACK PEARLS^{®}, ELFTEX^{®}, VULCAN^{®}, MOGUL^{®}, MONARCH^{®}, EMPEROR^{®}, and REGAL^{®}, etc., but is not limited thereto.

For example, the porous carbon-based carrier may have a pore volume of 0.1 cm³/g to 1.5 cm³/g, or 0.3 cm³/g to 1.5 cm³/g, or 0.6 cm³/g to 1.5 cm³/g.

Here, in the carbon-based carrier, the carbon may have a volume ratio of mesopores with a pore size of 2 nm to 50 nm among the total pores of 50% or more. Preferably, in the carbon carrier, the carbon may have a volume ratio of mesopores among the total pores of 70% or more, and more preferably, in the carbon carrier, the carbon may have a volume ratio of mesopores among the total pores of 75% or more.

At this time, if the volume ratio of mesopores is less than 50%, there may be a problem with the microscopic mass transfer rate of reactants and products within the carbon carrier, and if the average pore size is greater than 50 nm, there may be a problem with the weak physical strength of the carrier, so the above range is suitable.

In addition, the carbon-based carrier comprises ordered mesoporous carbon (OMC) comprising a BET specific surface area in the range of 100 m²/g to 1,500 m²/g. Preferably, the carbon may comprise ordered mesoporous carbon (OMC) comprising a specific surface area (BET) in the range of 200 m²/g to 1,000 m²/g.

At this time, if the specific surface area of the carbon-based carrier is less than 100 m²/g, there may be a problem in achieving high dispersion of active metal (Ru, Sn), and if the specific surface area of the carbon exceeds 1,500 m²/g, there may be a problem of a decrease in the ratio of mesopores, so the above range is suitable.

Meanwhile, the hydrogenation catalyst may have a BET specific surface area of 100 m²/g to 1,500 m²/g, or 200 m²/g to 1,500 m²/g, or 350 m²/g to 800 m²/g, or 550 m²/g to 670 m²/g.

In addition, the hydrogenation catalyst may have an average pore diameter of 2.0 nm to 5.5 nm, or 2.5 nm to 5.0 nm, or 3.0 nm to 4.8 nm, or 4.0 nm to 4.5 nm.

The hydrogenation catalyst according to an embodiment of the present disclosures, by comprising ruthenium and tin, which are catalytically active metal(s), within a specific range and forming a homogeneous alloy status of ruthenium and tin, can effectively convert a carboxylic acid group, an aldehyde group, or a ketone group directly to an alcohol group, for example, a primary alcohol, by their hydrogenation without esterification, and in such a hydrogenation reaction, it can achieve high selectivity and conversion rate under relatively low pressure conditions without excessive reaction time or high temperature conditions.

### Process for Preparing a Hydrogenation Catalyst

Accordingly, according to another embodiment of the present disclosures, a process for preparing the above-described hydrogenation catalyst is provided.

Specifically, the process for preparing a hydrogenation catalyst comprises:
preparing a metal precursor solution by dissolving one or more precursor compounds comprising ruthenium as catalytically active metal(s) and one or more precursor compounds comprising tin as catalytically active metal(s) in an acidic aqueous solution; and
performing a reduction treatment in the presence of hydrogen gas after drying the metal precursor solution or a mixture comprising it;
wherein the content of ruthenium is 30 wt% or more and less than 55 wt% and the content of tin is greater than 45 wt% and 70 wt% or less, based on the total weight of ruthenium and tin, in the metal precursor solution.

In particular, the precursor compound comprising ruthenium as catalytically active metal(s) is at least one selected from the group consisting of ruthenium metal, ruthenium chloride, ruthenium nitrate, acetylacetonatoruthenium, ruthenium carbonyl, ruthenium oxalate, and ruthenium nitrosyl nitrate.

In addition, the precursor compound comprising tin as catalytically active metal(s) is at least one selected from the group consisting of tin(II) chloride, sodium stannate, tin(II) acetate, tin fluoride, and tin iodide.

In the process for preparing a hydrogenation catalyst according to the present disclosures, an acidic aqueous solution is used to completely dissolve the precursor compound comprising ruthenium and tin, or a mixture thereof, thereby completely ionizing the precursor compound to prepare a metal precursor solution. By using such an acidic aqueous solution, ruthenium and tin do not solidify in a separated state, but can form an alloy in the form of a solid solution. Due to this characteristic, a broad and symmetrical hydrogen adsorption peak can be confirmed in the hydrogen adsorption curve graph for the hydrogenation catalyst according to an embodiment of the present disclosures. As a result, the hydrogenation catalyst according to an embodiment of the present disclosures can achieve superior hydrogenation reaction performance without additionally using expensive Pt and the like, and also has the excellent effect that no leaching of Sn occurs during the reaction.

However, if an acidic aqueous solution is not used in preparing the metal precursor solution, Sn and Ru are supported in a segregated state, which may lead to a decrease in catalytic activity even if the same content of metal precursor compound is used. In the hydrogen adsorption curve graph for the finally prepared catalyst, independent hydrogen adsorption peaks of Ru and Sn occur, and the reduction mechanism of the carboxylic acid group on the composite metal (Ru-Sn) phase does not proceed properly, making it inefficient for such catalytic reactions.

Such an acidic aqueous solution may comprise one or more of hydrochloric acid, nitric acid, and acetic acid, and an acidic aqueous solution with a concentration of 0.1 wt% to 10 wt% may be used to sufficiently dissolve the precursor compound or a mixture thereof. For example, deionized water comprising 1 wt% to 5 wt% hydrochloric acid may be used as the acidic aqueous solution. In the process for preparing a hydrogenation catalyst, if an excessive amount of acidic aqueous solution is used, corrosive gases such as HCl may be excessively generated during the subsequent reduction treatment process, which may cause corrosion of the reactor or process and act as a catalyst poison. Therefore, it is preferable to use the minimum content within the ranges described above.

In addition, in the metal precursor solution, the ruthenium content is 30 wt% or more and less than 55 wt% based on the total weight of ruthenium and tin, and the tin content is greater than 45 wt% and 70 wt% or less based on the total weight of ruthenium and tin.

In the metal precursor solution, the goal is to form a homogeneous alloy status of ruthenium and tin in the final composite metal catalyst, while comprising ruthenium and tin, which are catalytically active metal(s), within specific optimized ranges.

Specifically, the ruthenium content in the metal precursor solution may be comprised to be 1 part by weight or more and 11 parts by weight or less based on the total weight of the produced catalyst. More specifically, the ruthenium content in the metal precursor solution may be comprised to be 1.5 parts by weight or more and 10.5 parts by weight or less, or 2 parts by weight or more and 10 parts by weight or less, or 3.5 parts by weight or more and 9.5 parts by weight or less, or 4.5 parts by weight or more and 9 parts by weight or less, or 4.8 parts by weight or more and 8 parts by weight or less, or 5.0 parts by weight or more and 7.5 parts by weight or less, or 5.1 parts by weight or more and 7 parts by weight or less based on the total weight of the produced catalyst. In particular, when ruthenium is comprised in a high content, i.e., when ruthenium is comprised in an amount exceeding 11 parts by weight, there is a disadvantage of an increased catalyst price. In addition, there may be a problem of additional process costs due to a decrease in the selectivity of the target product as the size of the active metal comprising ruthenium and tin, which constitute the catalyst, increases. Furthermore, when ruthenium is comprised in an amount less than 1 part by weight, there may be a problem of decreased conversion efficiency and thus reduced production efficiency of a compound comprising an alcohol group, for example, 1,4-butanediol (BDO), in the hydrogenation reaction.

And, the tin content in the metal precursor solution may be comprised to be 1 part by weight to 11 parts by weight, or 2 parts by weight to 10.8 parts by weight, or 4.5 parts by weight to 10.5 parts by weight, or 5 parts by weight to 10.2 parts by weight, or 5.5 parts by weight to 10 parts by weight, or 5.9 parts by weight to 9.8 parts by weight based on the total weight of the produced catalyst.

Also, the tin content in the metal precursor solution may be comprised in a weight ratio of 0.8 to 2.67, or 0.9 to 2.5, or 1.0 to 2.4, or 1.1 to 2.3 relative to the ruthenium content on a weight basis. If the weight ratio is above the ranges described above, instead of an alloy of ruthenium and tin, independent metal forms exist, which reduces efficiency in the selective reduction reaction of the carbonyl group, and may lead to a decrease in conversion efficiency as the conversion proceeds to intermediate esterified compounds, such as gamma-butyrolactone (GBL), or ether compounds, such as tetrahydrofuran (THF), rather than to the target product, a compound comprising an alcohol group, for example, 1,4-butanediol (BDO).

In the metal precursor solution, the more specific content range and weight ratio of ruthenium and tin, which are catalytically active metal(s), may be applied at a level that enables the finally prepared composite metal catalyst to achieve the ranges previously described in relation to the hydrogenation catalyst, and specific details related thereto are omitted.

Meanwhile, the process for preparing a hydrogenation catalyst according to the present disclosures is characterized in that, in terms of enabling ruthenium and tin comprised in the catalyst to be included within specific optimized ranges as described above, Ru and Sn precursors are prepared by supporting them through an impregnation method, for example, an incipient wetness impregnation method, followed by drying and reduction.

Specifically, the process for preparing a hydrogenation catalyst may further comprise a step of mixing the metal precursor solution with a porous carbon-based carrier after preparing the metal precursor solution as described above. This mixing step may be part of the step of supporting ruthenium and tin as the catalytically active metal(s) on the porous carbon-based carrier.

The specific types and physical property ranges of the porous carbon-based carrier may be as described above, and thus a detailed description thereof is omitted.

In particular, the metal precursor solution or a mixture comprising it may be dried under temperature conditions of 323 K to 473 K, and then subjected to reduction treatment, i.e., reduction reaction in the presence of hydrogen gas under temperature conditions of 473 K to 773 K.

In addition, after the reduction treatment, the process process for preparing a hydrogenation catalyst of the present disclosures may further comprise a step of a step of passivating a reduction product obtained from the reduction treatment to form a passivation layer.

The passivating step is to passivate the reduction product with a nitrogen mixed gas comprising 0.1 to 20 volume% oxygen.

### Hydrogenation Method

According to further another embodiment of the present disclosures, a hydrogenation method is provided for performing a hydrogenation reaction using the above-described catalyst.

The hydrogenation method according to the present disclosures is characterized by performing a hydrogenation reaction that converts a carboxylic acid group, an aldehyde group, or a ketone group into an an alcohol group, in the presence of the hydrogenation catalyst previously described.

Specifically, in the hydrogenation reaction, the reaction pressure may be 50 bar to 150 bar, the reaction temperature may be 410 K to 560 K, and the reaction time may be 0.5 hours to 10 hours.

Meanwhile, the hydrogenation reaction may be performed using a carboxylic acid compound comprising a carboxylic acid group is at least one selected from the group consisting of oxalic acid, malonic acid, succinic acid, glutaric acid, adipic acid, pimelic acid, suberic acid, azelaic acid, sebacic acid, phthalic acid, isophthalic acid, terephthalic acid, formic acid, acetic acid, caproic acid, caprylic acid, lauric acid, myristic acid, palmitic acid, stearic acid, isostearic acid, oleic acid, maleic acid, cyclohexanecarboxylic acid, benzoic acid, and their anhydrides. Specifically, the carboxylic acid compound comprising a carboxylic acid functional group may be one or more species or two or more species selected from the group consisting of succinic acid, succinic anhydride, maleic acid, and maleic anhydride.

In addition, the hydrogenation reaction may be performed using an aldehyde compound comprising an aldehyde group is at least one selected from the group consisting of formaldehyde, propionaldehyde, n-butyraldehyde, isobutyraldehyde, valeraldehyde, 2-methylbutyraldehyde, 3-methylbutyraldehyde, 2,2-dimethylpropionaldehyde, capronaldehyde, 2-methylvaleraldehyde, 3-methylvaleraldehyde, 4-methylvaleraldehyde, 2-ethylbutyraldehyde, 2,2-dimethylbutyraldehyde, 3,3-dimethylbutyraldehyde, caprylaldehyde, caprinaldehyde, and glutaraldehyde. Specifically, the aldehyde compound comprising an aldehyde group may be n-butyraldehyde.

Further, the hydrogenation reaction may be performed using a ketone compound comprising a ketone group is at least one selected from the group consisting of acetone, butanone, pentanone, hexanone, cyclohexanone, and acetophenone. Specifically, the ketone compound comprising a ketone group may be butanone.

Meanwhile, the hydrogenation reaction may have a conversion rate represented by the following Formula 1 of 70% or more, or 70% or more to 100%. Conversion rate (%) = Moles of reactant consumed/Moles of reactant supplied x 100.

In Formula 1, the moles of reactant supplied represent the moles of the compound comprising a carboxylic acid group, an aldehyde group, or a ketone group supplied to the hydrogenation reaction, and the moles of reactant consumed represents the moles of the compound comprising a carboxylic acid group, an aldehyde group, or a ketone group consumed in the hydrogenation reaction.

The conversion rate is a value calculated as the molar ratio of the reactant consumed in the hydrogenation reaction relative to the reactant comprising a carboxylic acid group, an aldehyde group, or a ketone group supplied to the hydrogenation reaction when obtaining a compound comprising an alcohol group through the hydrogenation reaction. The conversion rate may be preferably 90% or more, or 90% or more to 99.9%.

In addition, the hydrogenation reaction may have a selectivity represented by the following Formula 2 of 50% or more, or 50% or more to 90%. Selectivity (%) = Moles of compound comprising alcohol group/Total moles of product x 100.

In Formula 2, the total moles of product represent the total moles of product generated from the hydrogenation reaction, and the moles of compound comprising an alcohol group represents the moles of the compound comprising an alcohol group converted from the carboxylic acid group, aldehyde group, or ketone group among the products generated from the hydrogenation reaction.

The selectivity is a value calculated as the molar ratio of the compound comprising an alcohol group, which corresponds to the target compound of the present disclosures, among the products generated through the hydrogenation reaction. The selectivity may be preferably 55% or more, or 55% or more to 70%.

In addition, the hydrogenation reaction may have a yield represented by the following Formula 3 of 50% or more, or 50% or more to 90%. Yield (%) = Conversion rate (%) of Formula 1 * Selectivity (%) of Formula 2 / 100.

The yield is a value calculated as the yield of the compound comprising an alcohol group generated through the hydrogenation reaction. The yield may be preferably 55% or more, or 55% or more to 70%.

In particular, the hydrogenation catalyst according to an embodiment of the present disclosures can have excellent effects of achieving high selectivity and conversion rate as described above, and securing alcohol compounds in high yield, in a hydrogenation reaction that converts a carboxylic acid group, an aldehyde group, or a ketone group into an alcohol group, under relatively low pressure conditions without excessive reaction time or high temperature conditions.

Hereinafter, the present invention will be described in more detail by the following exemplary embodiments. However, the following exemplary embodiments are only for illustrating the present invention, and the scope of the present invention is not limited to these exemplary embodiments.

### [EXAMPLES]

### Example 1

Activated carbon (pore volume: 0.65 cm³/g) was used as the porous carbon carrier. Here, the porous carbon carrier was prepared by drying under a temperature of 373 K (kelvin temperature) for 24 hours in a drying oven to remove residual moisture absorbed in the carbon carrier.

In addition, an acidic aqueous solution comprising hydrochloric acid was used to completely dissolve about 0.65 g of tin(II) chloride, SnCl₂·2H₂O. At this time, the acidic aqueous solution was prepared by adjusting the total concentration to 1 wt% using 35% grade of hydrochloric acid (Reagent-Grade product). About 0.03 g of ruthenium chloride, RuCl₃·3H₂O, of which ruthenium metal was corresponding to about 80 wt% of the tin metal, was mixed with the tin precursor solution and completely dissolved for about 3 hours. Here, the weight ratio of ruthenium/(ruthenium+tin) in the metal precursor solution was prepared to be 44.4 wt%.

The metal precursor solution obtained from the above preparation was uniformly mixed with the porous carbon carrier using a mortar and pestle. Thereafter, the well-mixed metal-carbon mixture was placed in a drying oven set to a temperature of 373 K and dried for 12 hours or more. Then, the dried metal-carbon mixture was reduced under hydrogen gas flow at a temperature of 623 K for about 3 hours, to prepare a hydrogenation catalyst in a powder phase. After reduction, the powdered catalyst was passivated for 3 hours using a nitrogen mixed gas comprising 1% oxygen to prepare the passivated catalyst. Then, the catalyst activity test was carried out using the obtained powdered hydrogenation catalyst, i.e., the passivated catalyst.

For the powdered hydrogenation catalyst thus prepared in Example 1, i.e., the passivated catalyst, the ratio of the amount of hydrogen adsorption at 523 K or less to the total amount of hydrogen adsorption of the passivated catalyst was measured by H₂-TPR analysis according to the method described below.

Specifically, Hydrogen-Temperature-Programed Reduction (H₂-TPR) analysis is an analytical method for evaluating the amount of hydrogen adsorption adsorbed by the metal at a specific reaction temperature, and the detailed measurement method is as follows.

### <H₂-TPR Analysis Method>

### - Pre-treatment: Catalyst pre-treatment and TCD signal stabilization

① Flow inert gas (Ar or N₂) through a layer comprising the catalyst and heat to 373 K at a rate of 5 K/min;
② Maintain inert gas flow through the catalyst layer at 373 K for 30 minutes;
③ Cool to 323 K while flowing inert gas through the catalyst layer;
④ Stabilize the TCD signal by flowing a mixture of inert gas and hydrogen through the catalyst layer for 60 minutes. At this time, the volume ratio of inert gas to H2 was fixed at 1:0.05.

### - H₂-TPR Analysis

① Analytical gas: H₂/Ar 5% mixed gas at 30 sccm (standard cubic centimeter per minute)
② Heat to 1073 K at a rate of 5 K/min.

As described above, the hydrogen adsorption curve of the passivation layer, which comprises the powdered hydrogenation catalyst of Example 1, i.e., the passivated catalyst, is shown in FIG. 1, which is analyzed by H₂-TPR for the powdered hydrogenation catalyst of Example 1, i.e., the passivated catalyst.

In FIG. 1, the area corresponding to the total amount of hydrogen adsorption in moles adsorbed by the passivation layer, which is surrounded by the H₂-TPR curve and baseline from the initial temperature of 333 K to the metal reduction temperature of 973 K, is defined as A1. The area corresponding to the amount of hydrogen adsorption in moles within the hydrogenation reaction temperature range, which is surrounded by the H₂-TPR curve and baseline from the initial temperature of 333 K to the hydrogenation reaction temperature of 523 K, is defined as A2. Then, the ratio (mol%) of A2 to A1 is shown in Table 1. That is, the A2/A1 measured in Example 1 is 94.5 mol%.

In addition, the metal content comprised within the catalyst was analyzed using inductively coupled plasma atomic emission spectrometry (ICP-AES). The specific surface area and average pore size of the catalyst were measured using nitrogen adsorption-desorption analysis.

Therefore, in the powdered hydrogenation catalyst of Example 1, i.e., the passivated catalyst, the ruthenium content was measured to be 51600 ppm by weight of the total catalyst, and the tin content was measured to be 59120 ppm by weight of the total catalyst. In addition, the size of the catalytically active metal was about 3.5 nm to 5.5 nm, and the average particle size was about 4.5 nm, which are measured by transmission electron microscopy (TEM). Further, the BET specific surface area of the powdered hydrogenation catalyst was measured to be 603 m²/g. The average pore diameter of the powdered hydrogenation catalyst was measured to be 4.1 nm. The specific physical property measurement results for the powdered hydrogenation catalyst of Example 1 are shown in Table 1 below.

### Example 2

A powdered hydrogenation catalyst of Example 2 was prepared by performing the same processes of drying, reduction, and passivating as in Example 1, except that the weight ratio of ruthenium/(ruthenium+tin), i.e., Ru/Ru+Sn, in the metal precursor solution was changed to 31 wt% and uniformly mixed with the porous carbon carrier.

The hydrogen adsorption curve of the passivation layer comprising the powdered hydrogenation catalyst, i.e., the passivated catalyst, prepared in Example 2, which was analyzed by H₂-TPR using the method described above, is shown in FIG. 1.

In addition, physical properties were evaluated for the powdered hydrogenation catalyst of Example 2, i.e., the passivated catalyst, by the same method as in Example 1. Specifically, in the powdered hydrogenation catalyst of Example 2, the ruthenium content was measured to be 52480 ppm by weight of the total catalyst, and the tin content was measured to be 117700 ppm by weight of the total catalyst. The size of the catalytically active metal was about 5 nm to 10 nm, and the average particle size was about 7.5 nm, which are measured by transmission electron microscopy (TEM). Further, the BET specific surface area of the powdered hydrogenation catalyst was measured to be 573 m²/g. The average pore diameter of the powdered hydrogenation catalyst was measured to be 4.3 nm. The specific physical property measurement results for the powdered hydrogenation catalyst of Example 2 are shown in Table 1 below.

### Comparative Example 1

A powdered hydrogenation catalyst of Comparative Example 1 was prepared by performing the same processes of drying, reduction, and passivating as in Example 1, except that the weight ratio of ruthenium/(ruthenium+tin), i.e., Ru/Ru+Sn, in the metal precursor solution was changed to 24 wt% and uniformly mixed with the porous carbon carrier.

The hydrogen adsorption curve of the passivation layer comprising the powdered hydrogenation catalyst, i.e., the passivated catalyst, prepared in Comparative Example 1, which was analyzed by H₂-TPR using the method described above, is shown in FIG. 1.

In addition, physical properties were evaluated for the powdered hydrogenation catalyst of Comparative Example 1, i.e., the passivated catalyst, by the same method as in Example 1, and the measurement results are shown in Table 1 below.

### Comparative Example 2

A powdered hydrogenation catalyst of Comparative Example 2 was prepared by performing the same processes of drying, reduction, and passivating as in Example 1, except that the weight ratio of ruthenium/(ruthenium+tin), i.e., Ru/Ru+Sn, in the metal precursor solution was changed to 100 wt% in Example 1 and uniformly mixed with the porous carbon carrier.

The hydrogen adsorption curve of the passivation layer comprising the powdered hydrogenation catalyst, i.e., the passivated catalyst, prepared in Comparative Example 2, which was analyzed by H₂-TPR using the method described above, is shown in FIG. 1.

In addition, physical properties were evaluated for the powdered hydrogenation catalyst of Comparative Example 2, i.e., the passivated catalyst, by the same method as in Example 1, and the measurement results are shown in Table 1 below.

### Comparative Example 3

A powdered hydrogenation catalyst of Comparative Example 3 was prepared by performing the same processes of drying, reduction, and passivating as in Example 1, except that ruthenium metal was added to achieve a ruthenium/(ruthenium+tin) weight ratio, i.e., Ru/Ru+Sn, of 47 wt% in the metal precursor solution, using an aqueous solution not comprising hydrochloric acid, i.e., deionized water, instead of an acidic aqueous solution of Example 1, to prepare the metal precursor solution. Thereafter, it was uniformly mixed with the porous carbon carrier to prepare a powdered hydrogenation catalyst of Comparative Example 3.

The hydrogen adsorption curve of the passivation layer comprising the powdered hydrogenation catalyst, i.e., the passivated catalyst, prepared in Comparative Example 3, which was analyzed by H₂-TPR using the method described above, is shown in FIG. 1.

In addition, physical properties were evaluated for the powdered hydrogenation catalyst of Comparative Example 3, i.e., the passivated catalyst, by the same method as in Example 1, and the measurement results are shown in Table 1 below.

Table 1 shows the weight ratio of metal components Ru and Sn in the metal precursor solution and the physical property for the powdered hydrogenation catalysts in Examples 1 to 2 (Exs. 1 to 2) and Comparative Examples 1 to 3 (Com. Exs. 1 to 3).

**[Table 1]**

| | | Ex. 1 | Ex. 2 | Com. Ex. 1 | Com. Ex. 2 | Com. Ex. 3 |
|---|---|---|---|---|---|---|
| metal precurs or solution | Ru / Ru+Sn (wt%) | 44.4 | 31 | 24 | 100 | 47 |
| | Sn / Ru+Sn (wt%) | 55.6 | 69 | 76 | 0 | 53 |
| | Solvent | HCl-comprisin g acidic aqueous solution | HCl-comprisin g acidic aqueous solution | HCl-comprisin g acidic aqueous solution | HCl-comprisin g acidic aqueous solution | HCl-uncompri sing aqueous solution |
| | A2 (mole) | 3.62 | 2.62 | 1.67 | 1.94 | 2.65 |
| | A1 (mole) | 3.82 | 2.91 | 2.58 | 0.90 | 4.25 |
| | A2/A1(mol%) | 94.5 | 87.7 | 64.9 | 68.3 | 58.6 |
| | Ru (ppm) | 51600 | 52480 | 51550 | 49950 | 51450 |
| | Sn (ppm) | 59120 | 117700 | 163900 | 0 | 61250 |
| Powder ed hydroge nation catalyst | Sn(ppm)/Ru( ppm) | 1.1457 | 2.2428 | 3.1794 | 0 | 1.1905 |
| | Catalyst's BET specific surface area (m²/g) | 603 | 573 | 514 | 687 | 617 |
| | Catalyst's average pore diameter (nm) | 4.1 | 4.3 | 4.2 | 4.4 | 4.6 |
| | Average particle size of active metal in catalyst (nm) | 4.5 | 7.5 | 13 | 3.5 | 5.5 |

In Table 1, the ratio of A2/A1 is the amount of hydrogen adsorption (A2) within the hydrogenation reaction temperature to the total amount of hydrogen adsorption (A1) of the hydrogenation catalyst. Specifically, the total amount of hydrogen adsorption (A1) was calculated by measuring the area corresponding to the total amount of hydrogen adsorption in mole from 333 K to 973 K by TPR analysis. The amount of hydrogen adsorption (A2) within the hydrogenation reaction temperature was calculated by measuring the area corresponding to the amount of hydrogen adsorption in moles from 333 K to 523 K by TPR analysis. Then, A2/A1 is calculated as the percentage ratio of A2 and A1.

### <Experimental Example 1>

A hydrogenation reaction of succinic acid was performed using each of the powdered hydrogenation catalysts of Examples 1 and 2 and Comparative Examples 1 to 3 by the following method. In the hydrogenation reaction of succinic acid, the succinic acid conversion rate (mol%), the selectivity (mol%) of major compounds in the product, and the yield (%) of 1,4-butanediol for each catalyst was measured by by the following method. The measurement results of are shown in Table 2 below.

Specifically, the hydrogenation reaction of succinic acid was performed using a batch reactor. First, 0.75 g of the powdered hydrogenation catalyst was placed into an autoclave reactor and succinic acid at a concentration of 2 wt% mixed with 150 mL of 1,4-dioxane solvent was added thereto. To minimize an oxidation reaction and other side reactions, the air in comprised inside the reactor was exhausted using hydrogen gas, which is the reaction medium. Then, after fastening the reactor, the internal temperature of the reactor was raised to 503 K while stirring at a speed of 300 rpm. After reaching the reaction temperature, hydrogen gas was pressurized to 90 bar, and then the stirring speed was increased to 1000 rpm. Thereafter, the hydrogenation reaction was carried out for 6 hours. At this time, samples of the reactants were collected at various times using the sampling port attached to the reactor.

The reaction products obtained after the reaction were then analyzed by gas chromatography (GC) equipped with columns (Agilent DB-FFAP, HP-5 column). Here, the conversion rate of succinic acid, the selectivity of the products, and the yield of 1,4-butanediol were calculated using Formulae 1a to 3a below. Succinic acid conversion rate (%) = Moles of succinic acid consumed / Moles of succinic acid supplied x 100. 1,4-butandiol Selectivity (%) = Moles of 1,4-butandiol in product / Total moles of product x 100. 1,4-butandiol Yield (%) = Succinic acid conversion rate (%) * 1,4-butandiol Selectivity (%) / 100.

**[Table 2]**

| | Conversion rate (mol%) | Selectivity (mol%) | | | 1,4-butandiol Yield (%) |
|---|---|---|---|---|---|
| | | gamma-butyrolacto ne | tetrahydrofu ran | 1,4-butandiol | |
| Ex. 1 | 99.5 | 22.7 | 1.7 | 61.4 | 61.1 |
| Ex. 2 | 99.9 | 46.3 | 6.5 | 55.9 | 55.8 |
| Com. Ex. 1 | 65 | 73.5 | 7.6 | 11.2 | 7.3 |
| Com. Ex. 2 | 43 | 95.8 | 2 | 0.5 | 0.2 |
| Com. Ex. 3 | 56 | 85.0 | 1.5 | 13.5 | 7.6 |

As shown in Table 2, when performing a hydrogenation reaction of succinic acid using the catalysts of Examples 1 to 2, which comprise ruthenium and tin as catalytically active metal(s) within a predetermined range and form a homogeneous alloy status of ruthenium and tin according to the present disclosures, 1,4-butanediol as an alcohol compound may be produced in high yield with high selectivity and simultaneously high conversion rate, minimizing conversion to gamma-butyrolactone (GBL) or tetrahydrofuran (THF), compared to Comparative Examples 1 to 3.

## Claims

1. A hydrogenation catalyst, comprising ruthenium and tin as catalytically active metal(s),
wherein the content of ruthenium is 30 wt% or more and less than 55 wt%, and the content of tin is greater than 45 wt% and 70 wt% or less, based on the total weight of ruthenium and tin, and
wherein the hydrogenation catalyst has a hydrogen adsorption ratio (A2/A1) of 70 mol% to 99 mol%, and the hydrogen adsorption ratio (A2/A1) is the amount of hydrogen adsorption (A2) at a temperature of 523 K or less of the hydrogenation catalyst to the total amount of hydrogen adsorption (A1) of the hydrogenation catalyst, as measured by a Hydrogen-Temperature-Programed Reduction (H₂-TPR) analysis.

2. The hydrogenation catalyst according to Claim 1,
wherein the ruthenium as the catalytically active metal is comprised in an amount of 1 part by weight or more and 11 parts by weight or less, based on the total weight of the hydrogenation catalyst,
the tin as the catalytically active metal is comprised in an amount of 1 part by weight or more and 12 parts by weight or less, based on the total weight of the hydrogenation catalyst, and
the tin is comprised in a weight ratio of 0.1 to 3.0 relative to the ruthenium on a weight basis in the catalytically active metal(s).

3. The hydrogenation catalyst according to Claim 1, wherein the catalytically active metal(s) are supported on a porous carbon-based carrier.

4. The hydrogenation catalyst according to Claim 3, wherein the porous carbon-based carrier comprises at least one selected from the group consisting of activated carbon, carbon black, graphite, graphene, ordered mesoporous carbon (OMC), and carbon nanotubes.

5. The hydrogenation catalyst according to Claim 3, wherein the porous carbon-based carrier has a pore volume of 0.1 cm³/g to 1.5 cm³/g.

6. The hydrogenation catalyst according to Claim 1, wherein the hydrogenation catalyst has a BET specific surface area of 100 m²/g to 1,500 m²/g.

7. The hydrogenation catalyst according to Claim 1, wherein the hydrogenation catalyst has an average pore diameter of 2.0 nm to 5.5 nm.

8. A process for preparing a hydrogenation catalyst, comprising:
preparing a metal precursor solution by dissolving one or more precursor compounds comprising ruthenium as catalytically active metal(s) and one or more precursor compounds comprising tin as catalytically active metal(s) in an acidic aqueous solution; and
performing a reduction treatment in the presence of hydrogen gas after drying the metal precursor solution or a mixture comprising it;
wherein the content of ruthenium is 30 wt% or more and less than 55 wt% and the content of tin is greater than 45 wt% and 70 wt% or less, based on the total weight of ruthenium and tin, in the metal precursor solution.

9. The process for preparing a hydrogenation catalyst according to Claim 8, wherein the precursor compound comprising ruthenium as catalytically active metal(s) is at least one selected from the group consisting of ruthenium metal, ruthenium chloride, ruthenium nitrate, acetylacetonatoruthenium, ruthenium carbonyl, ruthenium oxalate, and ruthenium nitrosyl nitrate.

10. The process for preparing a hydrogenation catalyst according to Claim 8, wherein the precursor compound comprising tin as catalytically active metal(s) is at least one selected from the group consisting of tin(II) chloride, sodium stannate, tin(II) acetate, tin fluoride, and tin iodide.

11. The process for preparing a hydrogenation catalyst according to Claim 8, further comprising a step of mixing the metal precursor solution with a porous carbon-based carrier after preparing the metal precursor solution.

12. The process for preparing a hydrogenation catalyst according to Claim 8, wherein the reduction treatment is performed under temperature conditions of 473 K to 773 K in the presence of hydrogen gas, after drying the metal precursor solution or a mixture comprising it under temperature conditions of 323 K to 473 K.

13. The process for preparing a hydrogenation catalyst according to Claim 8, further comprising a step of passivating a reduction product obtained from the reduction treatment to form a passivation layer, after performing the reduction treatment.

14. The process for preparing a hydrogenation catalyst according to Claim 13, wherein the passivating step is carried out to passivate the reduction product with a nitrogen mixed gas comprising 0.1 to 20 volume% oxygen.

15. A hydrogenation method, comprising:
performing a hydrogenation reaction to convert a carboxylic acid group, an aldehyde group, or a ketone group into an alcohol group, in the presence of the catalyst according to Claim 1.

16. The hydrogenation method according to Claim 15, wherein the hydrogenation reaction is performed under the reaction pressure of 50 bar to 150 bar and the reaction temperature of 410 K to 560 K in the reaction time of 0.5 hours to 10 hours.

17. The hydrogenation method according to Claim 15, wherein the hydrogenation reaction is performed using a carboxylic acid compound comprising a carboxylic acid group is at least one selected from the group consisting of oxalic acid, malonic acid, succinic acid, glutaric acid, adipic acid, pimelic acid, suberic acid, azelaic acid, sebacic acid, phthalic acid, isophthalic acid, terephthalic acid, formic acid, acetic acid, caproic acid, caprylic acid, lauric acid, myristic acid, palmitic acid, stearic acid, isostearyl acid, oleic acid, maleic acid, cyclohexanecarboxylic acid, benzoic acid, and their anhydrides.

18. The hydrogenation method according to Claim 15, wherein the hydrogenation reaction is performed using a aldehyde compound comprising an aldehyde group is at least one selected from the group consisting of formaldehyde, propionaldehyde, n-butyraldehyde, isobutyraldehyde, valeraldehyde, 2-methylbutyraldehyde, 3-methylbutyraldehyde, 2,2-dimethylpropionaldehyde, capronaldehyde, 2-methylvaleraldehyde, 3-methylvaleraldehyde, 4-methylvaleraldehyde, 2-ethyl butyraldehyde, 2,2-dimethylbutyraldehyde, 3,3-dimethylbutyraldehyde, caprylaldehyde, caprinaldehyde, and glutaraldehyde.

19. The hydrogenation method according to Claim 15, wherein the hydrogenation reaction is performed using a ketone compound comprising a ketone group is at least one selected from the group consisting of acetone, butanone, pentanone, hexanone, cyclohexanone, and acetophenone.
